(19) Europäisches Patentamt
European Patent Office
Office européen des brevets

(11) Publication number: **0 257 610 A2**

(12) # EUROPEAN PATENT APPLICATION

(21) Application number: **87112251.1**

(22) Date of filing: **24.08.87**

(51) Int. Cl.4: **C12P 7/22**, C12P 7/26, C07C 49/693, C07C 35/52, C07C 35/31, C07C 49/633, C07C 45/78, C07C 29/74, C07C 45/67, C07C 29/56, C07C 45/81

(30) Priority: **25.08.86 US 900029**

(43) Date of publication of application: **02.03.88 Bulletin 88/09**

(84) Designated Contracting States: **AT BE CH DE ES FR GB GR IT LI LU NL SE**

(71) Applicant: **SYNTEX (U.S.A.) INC.**
**3401 Hillview Avenue**
**Palo Alto, California 94304(US)**

(72) Inventor: **Kluge, Arthur F.**
**2900 Adeline Drive**
**Burlingame California 94010(US)**
Inventor: **Kertesz, Denis J.**
**459 Victory Avenue**
**Mountain View California 94043(US)**

(74) Representative: **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**D-8000 München 40(DE)**

(54) **Process and intermediates for the synthesis of the enantiomers of bicyclo(4.2.0)oct-2-en-7-one and derivatives and for the synthesis of bicyclo(4.2.0)octane derivatives.**

(57) The enantiomers of formulas

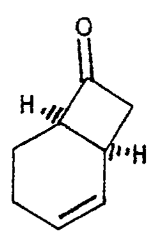

(1)

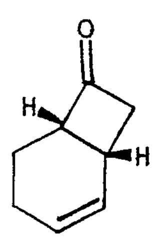

(2)

are prepared in a sequence starting from the racemic compound of formula

EP 0 257 610 A2

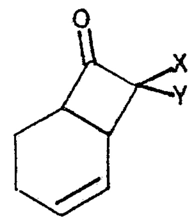

(3)

wherein X is hydrogen or bromo when Y is bromo, or X is chloro when Y is chloro.

The key step of this process involves a microbial reduction of the compound of formula (3) to give a ketone and an alcohol of high enantiomeric purity. The compounds of formulas (1) and (2) are intermediates for the preparation of certain bicyclo[4.2.0]octane derivatives.

# PROCESS AND INTERMEDIATES FOR THE SYNTHESIS OF THE ENANTIOMERS OF BICYCLO[4.2.O]OCT-2-EN-7-ONE AND DERIVATIVES AND FOR THE SYNTHESIS OF BICYCLO [4.2.0]OCTANE DERIVATIVES

This invention relates to a novel process for the synthesis of both enantiomers of bicyclo[4.2.0]oct-2-en-7-one and intermediates therefor and their application to the synthesis of certain bicyclo[4.2.0]octane derivatives, useful for treating cardiovascular disorders.

A method for the synthesis of certain bicyclo[4.2.0]octane derivatives, useful for treating cardiovascular disorders, was disclosed in European Patent Application No. 86104158.0, (EP-A-196 617). Three steps of this method involved separation of isomers from a diastereomeric mixture by (1) formation of a cobalt complex, (2) separation of this complex by chromatography and (3) conversion of the separated cobalt diastereomers back to the uncomplexed isomers.

It would be valuable to have a process for the preparation of the bicyclo[4.2.0]octane derivatives useful for treating cardiovascular disorders that did not involve the above three steps. A novel process has been discovered that achieves this end, by starting from resolved bicyclo[4.2.0]oct-2-en-7-one.

U.S. Patent No. 4130721 (Bundy et al.) describes the chemical resolution of a racemic mixture of bicyclo[4.2.0]oct-2-en-7-one. The aforementioned ketone is condensed with 1-ephedrine to afford a diastereomeric mixture of oxazolidines. The oxazolidines are separated by selective crystallization and an optically active isomer of the ketone is regenerated by acid hydrolysis of the oxazolidine moiety.

A microbiological separation of a racemic mixture of bicyclo[3.2.0]hept-2-en-6-one employing baker's yeast is disclosed by R.F. Newton et al., in the Journal of the Chemical Society, Chemical Communication, 1979, p. 908. The procedure suffers from two disadvantages: 1) The yeast reduction is not regioselective and produces a mixture of exo-and endo-hydroxyl groups which must be separated by chromatography, and 2) the reduction is not enantioselective and produces a 10:1 mixture of the desired to undersired isomer. The alcohol is then oxidized with Jones reagent to regenerate the enantiomerically enriched ketone.

H.G. Davies et al., Tetrahedron Letters, 27, 1986, p. 1093, discloses a procedure for the separation of an enantiomeric mixture of 7-endo-chlorobicyclo[3.2.0]hept-2-en-6-one using 3α,20β-hydroxysteriod dehydrogenase. The enzymatic reduction of the chloroketone provides exclusively the 6-endo-alcohol. However, the reduction is not enantioselective and yields a 10:1 mixture of the desired to undesired enantiomer. The alcohol is separated from the unreacted ketone and treated with an oxidant to provide the enantiomerically enriched ketone.

The present invention is a novel process for the preparation of the enantiomers represented by the formulas:

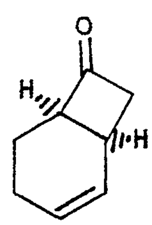

or

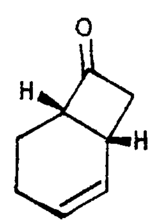

(1) (2)

from the compound

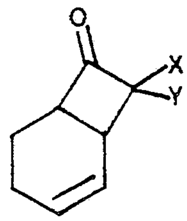

(3)

wherein X is hydrogen or bromo when Y is bromo, or X is chloro when Y is chloro.

Formula (3) includes two enantiomers, namely

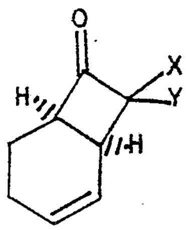

and

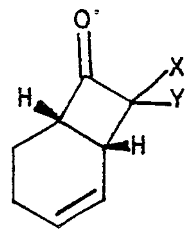

(4) (7)

More particularly, the present invention is a process for such conversion. Namely,

(a) microbial reduction of the compound of formula (3) to give a mixture represented by the formulas

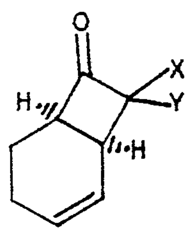

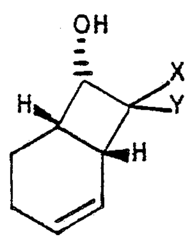

(4) (5)

(b) separation of the compounds of formulas (4) and (5),

(c) oxidation of the alcohol of formula (5) to the ketone represented by the formula

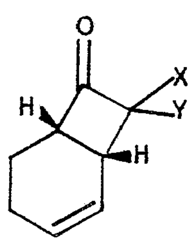

(7)

and (d) dehalogenation of the compounds of formulas (4) or (7) to give a compound represented by formula (1) or (2), respectively. (As described below, the sequence of some of the steps can be changed without seriously affecting the end result.) In particular, the key step of reducing the compound of formula (3) microbially gives the surprising result of rapidly reducing only one enantiomer of (3), namely (7), to give an alcohol of very high optical purity, the compound of formula (5), while leaving the other enantiomer, the compound of formula (4), unreacted and also of very high optical purity. This is an unexpectedly superior result when compared with attempts to separate the enantiomers conventionally, for example, by first preparing diastereomeric ketals or oxazolidines, by reaction with optically active reagents, of a compound of formula (3) and crystallizing the diastereomers apart in a tedious multi-recrystallization process. This is also an unexpectedly superior result when compared with the microbial reduction of other ketones, since the present microbial reduction is exceptionally rapid and, as only one enantiomer is reduced, the products are are easily separated. Another unexpectedly superior result is that the alcohols, the compounds of formulas (5) and (6) below, are solids and thus can be recrystallized to give maximum optical purity, leading to the desired products of formulas (1) or (2), in a 100% enantiomerically pure form.

Another aspect of the present invention is a second process for the preparation of both enantiomers represented by the formulas (1) and (2) as 100% optically pure isomers. Namely (a) microbial reduction of the compound of formula (3) to give a mixture represented by the formulas

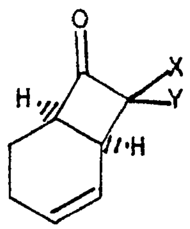

(4)

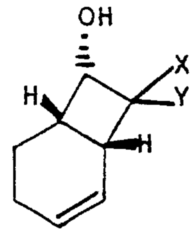

(5)

wherein X is hydrogen or bromo when Y is bromo, or X is chloro when Y is chloro,
(b) separation of the compounds of formulas (4) and (5),
(c) reduction of the compound of formula (4) to give the compound of the formula:

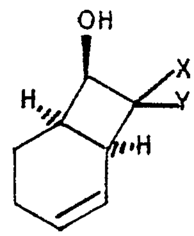

(6)

(d) optional purification of the compounds of formulas (6) or (5) recrystallization, (e) oxidation of the compounds of formulas (6) or (5) to the compounds of formula:

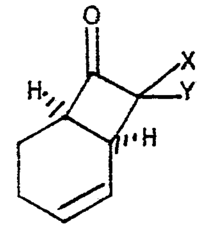

or

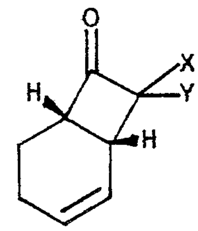

(4) (7)

respectively, and (f) dehalogenation of a compound of formula (4) or (7) to the compound of formula (1) or (2), respectively.

Yet another aspect of the present invention is a compound represented by the formula (4), (5), (6) or (7), wherein X is hydrogen or bromo when Y is bromo, or X is chloro when Y is chloro.

A further aspect of the present invention is a process for preparing bicyclo[4.2.0]octane derivatives represented by the formula:

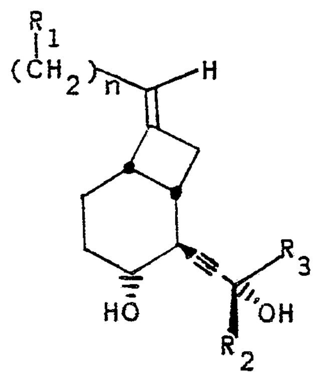

or

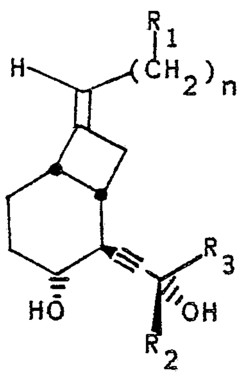

or

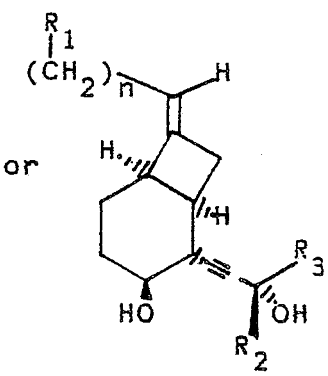

(8) (9) (10)

or the (E)-isomer of (10) and the pharmaceutically acceptable, non-toxic salts thereof wherein:

n is 2 or 3;

$R_1$ is $CH_2OH$, CHO, $CO_2R$ or $CO_2H$;

$R_2$ is hydrogen or methyl; and

$R_3$ is linear or branched alkyl having 5-10 carbon atoms,

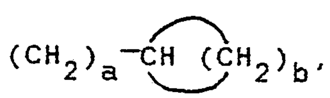, 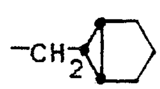, 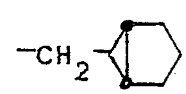, or $-(CH_2)_m$-phenyl

optionally substituted with lower alkyl, lower alkoxy, trifluoromethyl, or halogen,

in which

a is 0, 1 or 2;

b is 3-7;

m is 1 or 2; and

R is

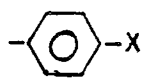

wherein X is

$-\overset{O}{\overset{\|}{C}}-CH_3$, $-NH\overset{O}{\overset{\|}{C}}CH_3$, $-\overset{O}{\overset{\|}{C}}-C_6H_5$, $-NH\overset{O}{\overset{\|}{C}}-C_6H_5$, or

$-NH\overset{O}{\overset{\|}{C}}N(R_4)_2$;

in which each $R_4$ is independently hydrogen or lower alkyl having 1-6 carbon atoms which process comprises:

(a) protecting the carbonyl group of an enantiomeric compound of the formula (1) or (2) to give a compound of the formula:

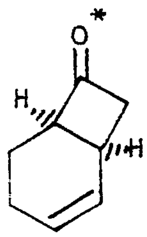

or

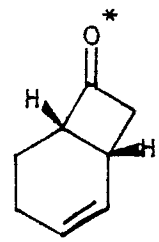

(11) (12)

wherein the asterisk signifies that the carbonyl group is protected,

(b) epoxidizing the compound of formula (11) or (12) to give compounds of the formulas:

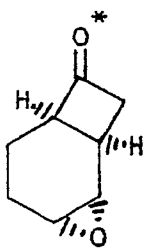

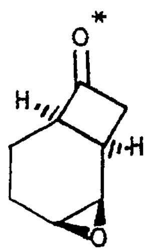

or

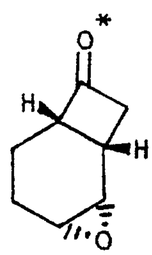

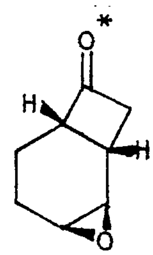

(13) (14) (15) (16)

as a mixture of (13) and (14) or a mixture of (15) and (16), respectively;

(c) treating the mixture of epoxides with a lithium acetylenic reagent to give a compound of the formula

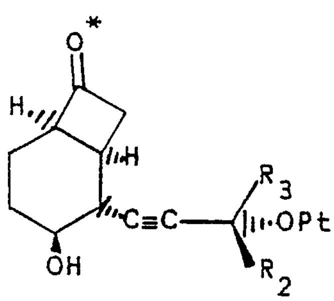

or

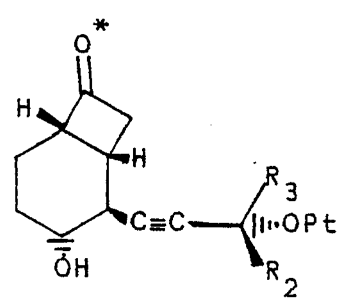

(18) (19)

wherein Pt is a protecting group;

(d) removing the protecting groups of formula (18) or (19) to give a compound of the formulas:

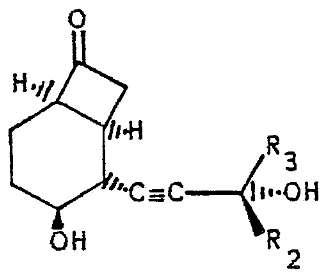 or 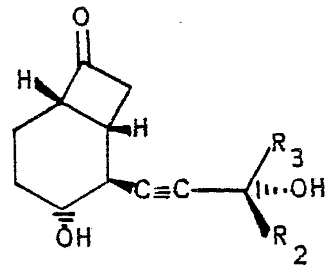

(20) (21)

(e) reacting the compounds of formulas (20) or (21) with a phosphorus ylid (Wittig reagent) to give a mixture of the componds of formulas (8) and (9) or (10) and the (E)-isomer corresponding to (10); and

(f) separating the individual isomers of (8) or (9) or (10) and is (E)-isomer, and optionally converting a compound of the formula (8) or (9) or (10) or its (E)-isomer wherein $R_1$ is COOH to a pharmaceutically acceptable, non-toxic salt thereof.

The preferred bicyclo[4.2.0]octane derivatives are compounds of the formula (8) or (10); more specifically preferred are the compounds of formula (8) or (10) wherein $R_1$ is COOH and the pharmaceutically acceptable, non-toxic salts thereof.

The compounds of the formula (8) or (9) or (10) or its (E)-isomer are useful for the treatment of cardiovascular disorders; in particular they are vasodilators, and inhibit accumulation of cholesterol in the vascular wall and in plasma. They are also potent inhibitors of the aggregation of platelets and the release from them of pro-coagulant and pro-atherosclerotic factors. Accordingly, these compounds are useful in treating and preventing cardiovascular disorders involving arteriosclerosis, thrombotic and vasospastic conditions. They also are useful antihypertensive and cholesterol lowering agents.

The compounds of this invention display the spectrum of activities associated with prostacyclin. However, in contrast to prostacyclin, whose therapeutic potential is severely compromised by its extreme chemical instability, the compounds of our invention retain high biological activity while displaying much greater chemical stability, a combination of attributes identifying them as promising agents for prophylactic and/or therapeutic use particularly in the treatment of cardiovascular dysfunction and disease. Many of these compounds are selective in their antithrombotic effect, and they achieve this therapeutic effect without substantially affecting blood pressure.

Yet a further aspect of the present invention is the use of the intermediates of the formulas (3), (4), (7), (5), (6), (1) and (2) for the preparation of bicyclo[4.2.0]octane derivatives.

## Definitions

The term "alkyl" refers to and includes saturated branched and straight chain hydrocarbon radicals containing the number of carbons indicated. Typical alkyl groups include methyl, ethyl, propyl, isopropyl, butyl, tertiary butyl, neopentyl, isopentyl, n-hexyl, n-octyl, n-nonyl, isodecyl, 6-methyldecyl.

"Cycloalkyl" as used herein means a saturated monocyclic hydrocarbon radical containing 3-8 carbon atoms, such as cyclopropyl, cyclobutyl, cyclopentyl, cyclohexyl, cycloheptyl and cyclooctyl.

The term "lower alkyl" refers to a branched or unbranched saturated hydrocarbon chain of 1-4 carbons, such as, for example, methyl, ethyl, n-propyl, i-butyl and the like.

The term "alkoxy" refers to the radical -O-alkyl wherein "alkyl" is as defined above. This includes radicals such as methoxy, ethoxy, 2-propoxy, n-butoxy, 3-pentoxy and the like.

"Lower alkoxy" means the group -OR wherein R is lower alkyl as herein defined.

"Halo" as used herein denotes fluoro, chloro, bromo, or iodo.

"Phenyl" as used herein encompasses all possible isomeric phenyl radicals optionally monosubstituted with a substituent selected from the group consisting of lower alkyl, lower alkoxy, hydroxy, trifluoromethyl and halogen.

"Optional" or "optionally" means that the subsequently described event or circumstance may or may nor occur, and that the description includes instances where said event or circumstance occurs and instances in which it does not. For example, "optionally substituted phenyl" means that the phenyl may or may not be substituted and that the description includes both unsubstituted phenyl and phenyl wherein there is substitution.

"Racemic" or "non-racemic" refers to mixtures of the enantiomers of a chiral molecule wherein the ratio of the amount of one enantiomer to the amount of its optical isomer is 1:1 or is not 1:1, respectively.

"Microbial reduction" is intended to encompass any reduction carried out by a micro-organism, or a preparation derived from a micro-organism, such as yeasts, yeast extracts, enzymes, dehydrogenases and the like.

"Bakers Yeast" is the common term for Saccharomyces cerevisiae.

"Pharmaceutically acceptable non-toxic salts" of the compounds of the formula (8) or (9) or (10) or its (E)-isomer are carboxylic acid salts obtained by reaction of the COOH moiety in formula (8) or (9) or (10) or its (E)-isomer with a suitable amine or inorganic base. Representative pharmaceutically acceptable bases are sodium hydroxide, potassium hydroxide, lithium hydroxide, ammonium hydroxide, calcium hydroxide, magnesium hydroxide, ferrous hydroxide, zinc hydroxide, copper hydroxide, manganous hydroxide, aluminum hydroxide, ferric hydroxide, manganic hydroxide, isopropylamine, trimethylamine, diethylamine, triethylamine, tripropylamine, ethanolamine, 2-dimethylaminoethanol, triethanolamine, 2-diethylaminoethanol, lysine, arginine, histidine, procaine, choline, betaine, ethylenediamine, glucosamine, methylglucamine, glucamine, theobromine, purines, piperazine, piperidine, N-ethylpiperidine, polyamine resins and the like.

"Protecting groups" means any suitable chemical group that is commonly used in the practice of organic chemistry to modify one or more of the major functional groups in a molecule -the hydroxyl, amino, carboxyl and carbonyl groups-for the purpose of selectively performing a chemical reaction at another reactive site in a multifunctional molecule. A protecting group is typically formed in a selective manner and is stable to subsequent reactions on the molecule and is selectively removed by reagents that do not attack the regenerated functional group. Suitable protecting groups for the hydroxyl group are: ethers including methyl ethers, substituted methyl ethers, substituted ethyl ethers, silyl ethers including trimethylsilyl, triethylsilyl, isopropyldimethylsilyl, t-butyldimethylsilyl, (triphenylmethyl)dimethylsilyl, t-butyldiphenylsilyl, and the like; esters including formate esters, acetate esters and their derivatives such as chloroacetate, trichloroacetate, trifluoroacetate, and the like, adamantoate, crotonate, benzoate and substituted benzoate derivatives, and the like; carbonates including methyl, ethyl, 2,2,2-trichloroethyl, isobutyl, vinyl, allyl, benzyl and derivatives such as p-methoxybenzyl, o-nitrobenzyl, and the like, carbamates including N-phenylcarbamate, N-imidazolylcarbamate, and the like, borate, N,N,N′,N′-tetramethylphosphororo-diamidate, 2,4-dinitrophenylsulfenate, and the like as well as protection for 1,2-and 1,3-diols including cyclic acetals and ketals, cyclic ortho esters and the like. Suitable protecting groups for the amino group are carbamates such as methyl carbamates and its derivatives like cyclopropylmethyl, diisopropylmethyl, 9-fluorenylmethyl carbamates and the like, substituted ethyl carbamates such as 2,2,2-trichloroethyl, 2-haloethyl carbamates, and the like, substituted propyl and isopropyl carbamates such as 1,1-dimethylpropynyl, 1-methyl-1-phenylethyl and derivatives, isobutyl, t-butyl carbamate, t-amyl carbamate, vinyl and allyl carbamate, phenyl and substituted phenyl carbamate, benzyl carbamate and derivatives such as p-methoxybenzyl, 3,5-dimethoxybenzyl, o-and p-nitrobenzyl, halobenzyl, and the like; amides and their derivatives such as N-acetyl and derivatives like N-dichloracetyl, N-trifluoroacetyl, and the like, substituted N-propionyl derivatives such as N-3-phenylpropionyl and derivatives, N-o-nitrocinnamoyl and the like, cyclic imide derivatives such as N-phthaloyl, N-2,3-diphenylmaleoyl, and the like; N-alkyl derivatives such as N-allyl and the like, amino acetal derivatives such as N-2,4-dinitrophenyl and the like, N-benzyl derivatives such as N-benzyl, N-o-nitrobenzyl, N-triphenylmethyl and the like, imine derivatives such as N,N′-isopropylidene, N-benzylidene and the like, enamine derivatives such as N-(5,5,-dimethyl-3-oxo-1-cyclohexenyl) and the like, N-heteroatom derivatives such as N-metal like N-borane, N-copper or N-zinc chelate and the like, N-N derivatives such as N-nitro, N-nitroso, N-oxide and the like, N-P derivatives such as N-diphenylphosphinyl, N-diphenyl phosphoryl and the like, N-S derivatives such as N-trimethylsilyl and the like, N-S derivatives such as N-sulfenyl like N-benzenesulfenyl, N-o-nitrobenzenesulfenyl and the like, N-sulfonyl derivatives such as N-benzenesulfonyl, N-2,4,6-trimethylebenzenesulfonyl and the like. Suitable protecting groups for the carboxyl group are esters such as methyl and substituted methyl like methoxymethyl, tetrahydropyranyl, methoxyethoxymethyl, phenacyl and derivatives, and the like; ethyl esters and 2-substituted ethyl esters such as 2,2,2-trichloroethyl, 2-(trimethylsilyl)ethyl, 2-methylthiomethyl, t-butyl, allyl, benzyl and the like; substituted benzyl esters such as triphenylmethyl, diphenylmethyl, o-nitrobenzyl, p-methoxybenzyl and the like; silyl esters such as trimethylsilyl, t-butyldimethylsilyl, i-propyldimethylsilyl and the like; activated esters such as S-t-butyl, S-2-pyridyl, N-hydroxyphthalimidoyl and the like; 2-alkyl-1,3-oxazolsines, 4-alkyl-5-oxo-1,3-oxazolidines, 5-alkyl-4-oxo-1,3-dioxalanes and the like; stannyl esters such as triethylstannyl, tri-n-butylstannyl and the like; amides and hydrazides such as N,N-dimethyl, pyrrolidinyl, hydrazides, N-phenylhydrazide and the like. Suitable protecting groups for the carbonyl group are acetals and ketals such as acyclic acetals and ketals such as dimethyl, diethyl, dibenzyl and the like; cyclic acetals and ketals such as 1,3-dioxanes like 5-methylene-1,3-dioxane and 1,3-dioxolanes such as 4-bromomethyl-1,3-dioxolane and the like; dithio acetals and ketals such as acyclic dithio acetals and ketals like S,S'-dimethyl, S,S'-diethyl, S,S'-diphenyl and the like and cyclic dithio acetals and ketals such as 1,3-dithiane, 1,3-dithiolane and the like; hemithio acetals and ketals such as acyclic hemithio acetals and ketals like O-trimethylsilyl-S-alkyl and cyclic hemithio acetals and ketals such as 1,3-oxathiolanes and the like; O-substituted cyanohydrinssuch as O-trimethylsilyl and the like; substituted hydrazones such as N,N-dimethyl and the like and oxime derivatives such as O-benzyl and the like; imines such as substituted methylene derivatives; cyclic derivatives such as oxazolidines, imidazolidines, thiazolidines and the like.

"Protecting" and "deprotecting" mean the formation and removal, respectively, of a protecting group according to the criteria defined above.

The numbering system for the bicyclo[4.2.0]octane system shown in the scheme illustration below is used in naming the intermediates and product compounds of the invention.

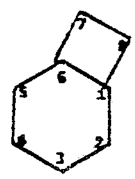

The absolute stereochemistry at carbons 1 and 6 are specified according to the Cahn-Ingold-Prelog R-S system. When the compound is a pure enantiomer, the stereochemistry at each chiral carbon is specified by either R or S. When a compound is a racemic mixture the stereochemistry at each chiral carbon is specified by either RS or SR, by reference to a single enantiomer of the racemate. In this manner relative stereochemistry is conveyed unambiguously.

The products of the reactions described herein can be isolated and purified by any suitable separation or purification procedure, such as, for example, filtration, extraction, crystallization, column chromatography, thin-layer chromatography, thick-layer chromatography, preparative low or high pressure liquid chromatography, distillation or a combination of these procedures. Specific illustrations are described in the Examples. However, other equivalent separation or purification procedures can be used.

Utility

The compounds of formula (1) and (2) prepared by the present process and their precursors described earlier are utilized in the preparation of certain bicyclo[4.2.0]octane derivatives, in particular the compounds of formulas (8), (9), (10) and the (E)-isomer of (10) and the pharmaceutically acceptable, non-toxic salts of these compounds. With respect to the preparation of the compounds of formula (8), (9), (10) and its (E)-isomer the following reaction conditions, reagents and protecting agents are preferred:

Step (a) ketalization of the compound of the formula (1) or (2) is performed at -20°C to 110°C with glycols and dithiols, in particular ethylene glycol;

Step (b) epoxidation of the compound of the formula (11) or (12) is performed at -40°C to 50°C with N-bromo amide derivatives, in particular N-bromoacetamide;

Step (c) the preferred lithium acetylenic reagent for treating the mixture of epoxides of the formula (13) and (14) or (15) and (16) has the formula

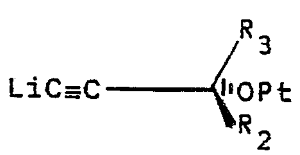

(17)

wherein $R_2$ and $R_3$ have the above meanings and Pt is a protecting group, in particular the t-butyldimethylsilyl group, and the reaction is performed at -100°C to about 0°C with a Lewis acid, in particular boron

trifluoride;

Step (d) removing the protecting groups (deprotecting) of the compound of the formula (18) or (19) under acidic conditions, in particular with sulfuric acid;

Step (e) the preferred phosphorus ylid (Wittig reagent) is of the formula

$$P'-\overset{\ominus}{C}H-(CH_2)_nR_1$$

wherein P' is a residue normally associated with olefination reactions, n is 2 or 3, and $R_1$ is as defined above;

Step (f) the preferred procedure for separating the individual isomers of (8) or (9) or (10) and its (E)-isomer is flash chromatography, and the optional conversion of these compounds wherein R is COOH to pharmaceutically acceptable salts is preferably performed by reaction with organic and inorganic bases at temperatures from 0°C to 75°C.

The preferred process for the preparation of the intermediates of formulas (1) and (2) is illustrated in more detail in the following reaction scheme.

REACTION SCHEME 1

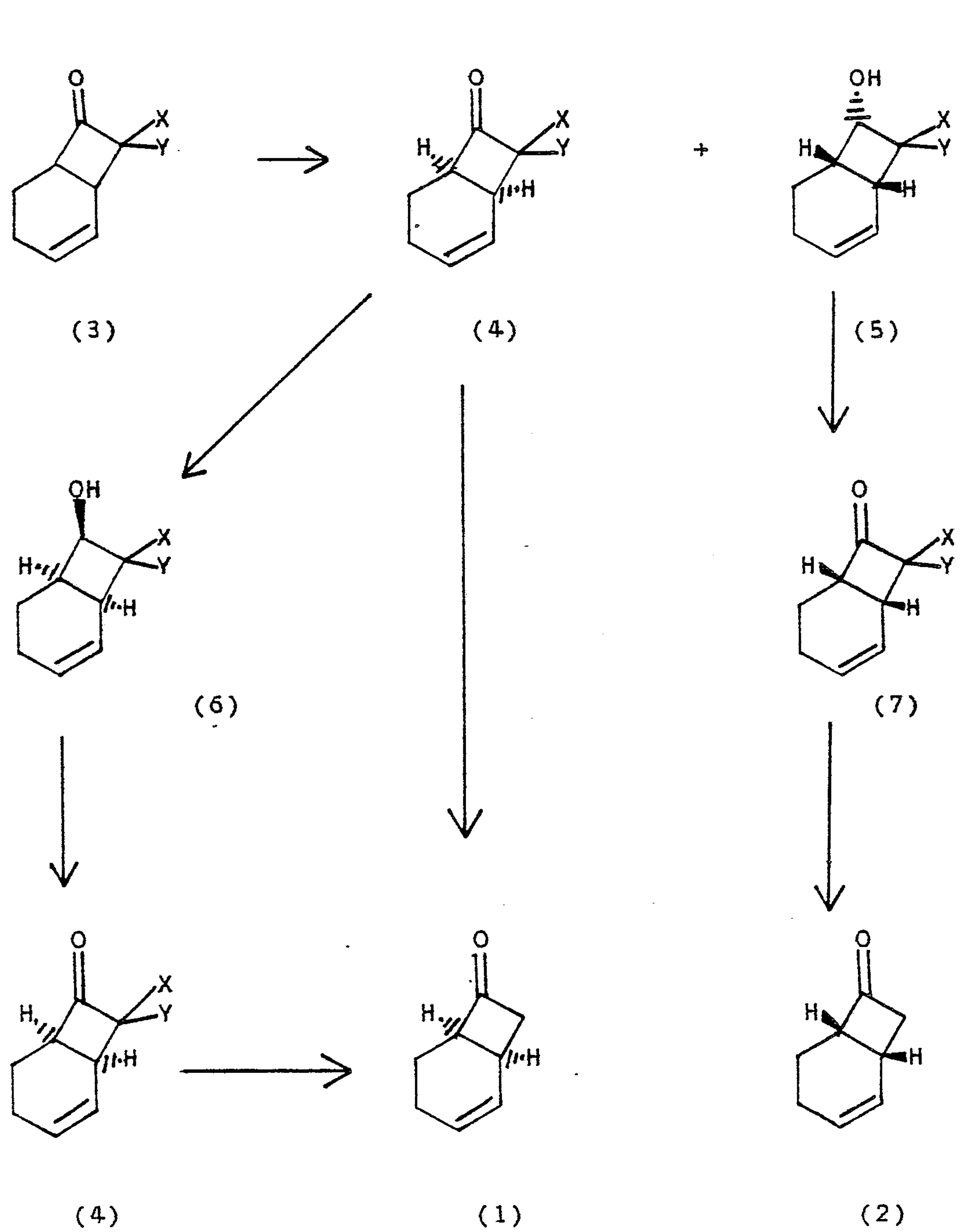

(3) (4) (5)

(6) (7)

(4) (1) (2)

In the first step, the racemic 8-monobromo or 8,8-dihalobicyclo[4.2.0]oct-2-en-7-one (3), preferably 8,8-dichlorobicyclo[4.2.0]oct-2-en-7-one, is treated with a micro-organism, preferably Baker's Yeast, optionally in the presence of a yeast nutrient and a sugar, such as glucose, dextrose or preferably sucrose. For every gram of ketone about 2-25 g, preferably about 8-15 g, of Baker's Yeast is used, plus about 0 g to 1.0 g, preferably about 0.7 g, of yeast nutrient, and about 0 g to 2.0 g, preferably about 0.5 g, of sucrose. The reaction is carried out in a yeast-compatible solvent such as a lower alcohol and water, such as about 2% to 10% ethanol, preferably about 5% ethanol, in water at a temperature of about 20°-40°C, preferably about 33°C, for about 15 minutes to 2 hours, preferably about 45 minutes. The ketone (4) and the alcohol (5) are isolated, separated and purified by conventional means, preferably by column chromatography.

The ketone of formula (4) may optionally be reduced to the alcohol of formula (6) by methods known per se to reduce the oxo functions while retaining the double bond between position 1 and 2, for example with a mild reducing agent then recrystallized from a suitable solvent and oxidized back to the compound of formula (4). This process acts as a purification procedure for the compound of formula (4), and results in a compound of 100% enantiomeric purity. For example, the ketone of formula (4) is reacted with about 1 to 10 molar equivalents, preferably about 2 to 4 molar equivalents, of sodium borohydride in a protic solvent such as water, ethanol or preferably methanol at a temperature of about 0-25°C, preferably about 5°C, for about 15 minutes to 4 hours, preferably about 1 hour, giving the compound of Formula (6), which is isolated and purified by conventional means, preferably recrystallization.

The alcohols of formula (5) or (6) are then converted to the ketone of formula (7) or (4), respectively, using an oxidizing agent appropriate to oxidize a secondary alcohol to a ketone such as potassium permanganate, ruthenium tetroxide, Moffatt oxidation, dimethylsulfoxide/acetic anhydride and modifications thereof, N-bromoacetamide and N-bromosuccinimide, and an aqueous solution of chromic acid and sulfuric acid (Jones reagent), sodium dichromate or an organic chromium reagent, preferably pyridinium chlorochromate. Typically, the ketone is reacted with about 1.5 to 4 molar equivalents, preferably about 3 molar equivalents, of pyridinium chlorochromate in the presence of about 4 to 10 molar equivalents, preferably about 6 molar equivalents, of magnesium sulfate in an inert solvent such as chloroform or preferably methylene chloride. The reaction is carried out at a temperature of about 30°-70°C, preferably about 45°C, for about 2 to 10 hours, preferably about 4 hours. When the reaction is substantially complete, the products are isolated and purified by conventional means.

The haloketones of formulas (4) or (7) are dehalogenated to the unsubstituted enantiomeric ketones of formulas (1) or (2), respectively, in a manner known per se, for example, with a mild dehalogenating agent, for example, tributyltin hydride, a zinc-copper couple or preferably zinc in acetic acid. The dehalogenation is effective at temperatures between room temperature up to 150°C. Typically, the dichloroketone is dissolved in an organic carboxylic acid, preferably acetic acid and reacted with about 2 to 10 molar equivalents, preferably about 5 molar equivalents, of zinc dust at a temperature of about 50°-100°C, preferably about 70°C, for about 20 minutes to 2 hours, preferably about 1 hour. A further quantity of zinc, about 2 molar equivalents, is then added and the mixture heated at about 70°-100°C, preferably about 75°C, for about 20 minutes to 2 hours, preferably about 1 hour. When the reaction is substantially complete the products of formula (1) or (2) are isolated and purified by conventional means.

The above reaction scheme demonstrates the preferred reaction sequence for the conversion of the compounds of formula (4) and (5) to the compounds of formula (1) and (2,) respectively. It is obvious that several alternative reaction pathways also arrive at the compounds of formula (1) and (2). Some alternative routes are illustrated below in Reaction Scheme 2, using hte compound of formula (5) where X and Y are chlorine as an example.

REACTION SCHEME 2

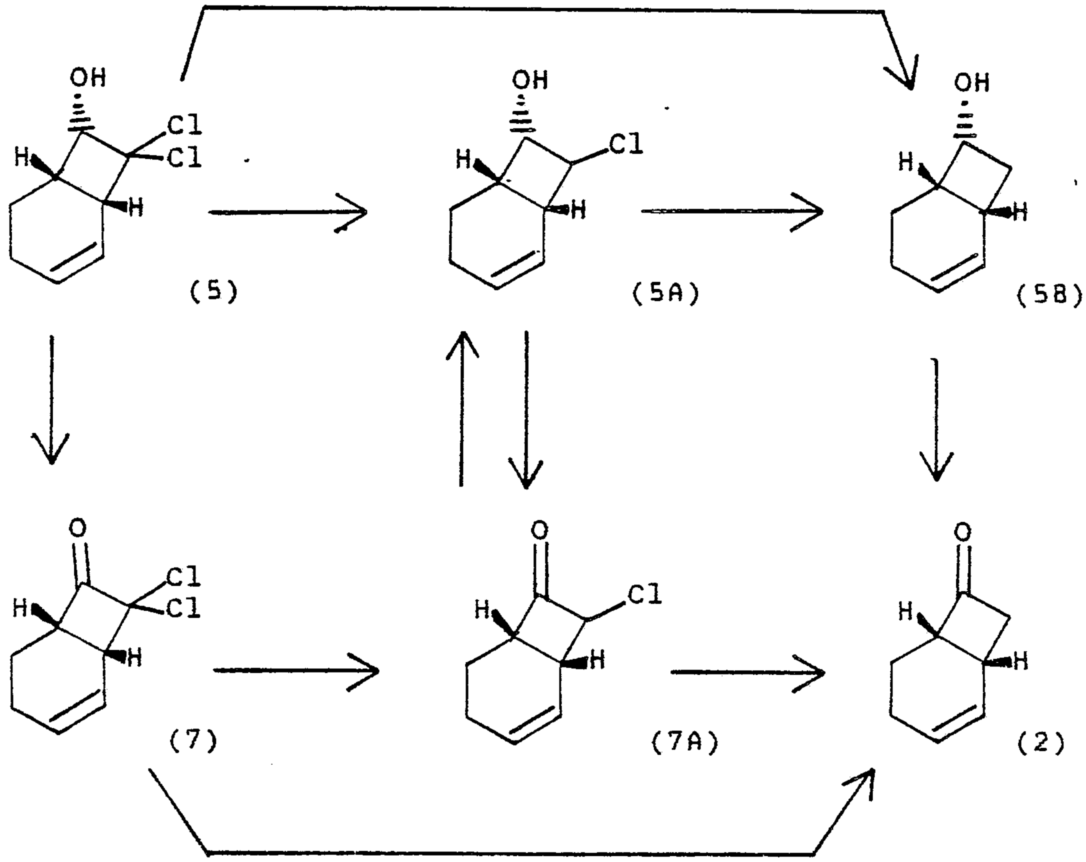

(5) (5A) (5B)

(7) (7A) (2)

For example, treatment of the compound of formula (5) with a dehalogenating agent, for example tri-n-butyltin hydride, for a brief period of time gives the monochloro compound of formula (5A). Treatment of the compounds of formula (5) or (5A) with a dehalogenating reagent, for example, tri-n-butyltin hydride at elevated temperatures and prolonged reaction time gives the compound of formula (5B) , which is then oxidized with a reagent suitable for oxidizing a secondary alcohol to a ketone as shown in Reaction Scheme 1 and as described above to give the desired compound of formula (2).

Furthermore, the compound of formula (5B), and protected forms thereof, can also be processed in accordance with the reaction sequences described herein.

The compound of formula (7), obtained by oxidation of the compound of formula (5) as shown in Reaction Scheme 1 above, may in the same manner be first converted to the monochloro compound of formula (7A) by treatment with a dehalogenating agent, for example zinc in acetic acid, for a short period of time at room temperature. Treatment of the compounds of formula (7) or (7A) under stronger dehalogenating conditions such as zinc in acetic acid at elevated temperatures, as shown in Reaction Scheme 1, gives the desired compound of formula (2). It should also be noted that the compounds of formula (5A) and (7A) are interconvertible by the above described standard oxidation and reduction reactions.

Similarly, starting with the compound of formula (4) wherein X and Y are chlorine, for example, and reducing it to the compound of formula (6), as shown in Reaction scheme 1, the same synthetic pathways as shown in Reaction Scheme 2 may be applied to arrive at the compound of formula (1). Similarly, the same reaction sequences apply for compounds of formula (4) and (5) where X and Y are bromo, or where X is hydrogen and Y is bromo.

## Preparation of Starting Materials

The compound of formula (3) where X and Y are each chloro or bromo, is made by reaction of 1,3-cyclohexadiene and the appropriate dihaloacetyl chloride. The reaction is discussed in more detail in Tetrahedron27:615, (1971), which is incorporated herein by reference.

The phosphorus ylides (Wittig reagents) are prepared by procedures well known in the chemical arts. For example, reaction of the appropriate ω-halocarboxylic acid with triphenylphosphine to give the corresponding triphenyl phosphonium salt, which is reacted with a base. The reaction is discussed in more detail in J. Org. Chem. 27, 3404 (1962), which is incorporated herein by reference.

The chiral acetylenic alcohols used to prepare the salt of formula (17) are prepared by reducing the appropriate acetylenic ketone with a chiral reducing agent, for example, (S)-8-isopinocamphenyl-9-borabicylco[3.3.1]nonane. Alternatively, the racemic acetylenic alcohols are converted to a hemiphthalate and the diastereomeric salts formed with an optically active amine separatged by recrystallization. These reactions are discussed in more detail in Patent Case 24880 (European Patent Application No. 86104158.0, European Patent Publication No. 196,617).

## Preferred Embodiments

One preferred embodiment of the present invention includes the process whereby the micro-organism is Baker's Yeast. A more preferred embodiment includes the process whereby X and Y are both chloro. Another more preferred embodiment includes the process whereby the oxidizing agent is pyridinium chlorochromate. Yet another more preferred embodiment includes the process whereby the dehalogenating agent is zinc in acetic acid.

A most preferred embodiment includes the process whereby the compounds of formula (5) and (6) are recrystallized to give 100% pure enantiomers.

In addition, the preferred intermediate of the present invention is the compound of the formula (2).

The following examples serve to illustrate the invention. They should not be construed as in any way narrowing or limiting the scope of the invention as claimed.

## Preparation 1

### Preparation of 3-cyclohexylprop-1-yn-3-ol

A rapid stream of acetylene was passed through a solution of 2M methyl magnesium bromide (100 ml) in THF until no more methane evolution was observed. 10 g of cyclohexanal was added at 0°C, stirred for 1/2 h and a saturated solution of $NH_4Cl$ was added. The organic product was isolated by extraction with ether. The ether solution was washed with water, brine, dried over $MgSO_4$ and evaporated to give a liquid, which was purified by distillation, to give 3-cyclohexylprop-1-yn-3-ol.

## Preparation 2

### Preparation of 1-Cyclohexyl-2-propyn-1-one

A solution of chromic acid was prepared by dissolving 106.88 g chromium trioxide in 400 ml water and then added 92 ml concentrated sulfuric acid. This solution is added in dropwise fashion over a 3 hr. period to an ice cooled, stirred solution of 120 g 3-cyclohexyl-1-propyn-3-ol in 175 ml acetone. The resulting mixture was diluted with 500 ml water and the product was extracted into 1 liter of diethyl ether. The ether extract was washed with 250 ml saturated sodium bisulfite solution and was dried over sodium sulfate. The diethyl ether was removed by distillation under nitrogen atmosphere and the resulting residue was purified by Kugelrohr distillation (65°C, 0.1 mm Hg) to give 84.9 of 1-cyclohexyl-2-propyn-1-one as an oil: MS m/z = 136 (M+) Caled. for $C_9H_{12}O$:C, 79.37; H, 8.88. Found:C, 79.24; H, 8.6.

## Preparation 3

Preparation of (S)-3-cyclohexyl-1-propyn-3-ol

A mixture of 1.6 liters 0.5 M 9-borabicyclo[3.3.1]nonane in tetrahydrofuran and 122.6 g (-)-α-pinene was heated at reflux under nitrogen for 4 hr., at which time the excess (-)-α-pinene and tetrahydrofuran were removed under vacuum to leave a thick oil. The contents of the flask were cooled to 0°C and 80 g of 1-cyclohexyl-2-propyn-1-one added with stirring. The resulting mixture was allowed to warm to 23°C and it was stirred at that temperature for 16 hr. Excess S-Alpine borane was destroyed by adding 44 ml propionaldehyde and stirring at 23°C for 1 hr. The liberated (-)-α-pinene was removed by vacuum distillation. The resulting mixture was diluted with 400 ml tetrahydrofuran followed by 30 ml 3N sodium hydroxide. To this stirred mixture was added in dropwise fashion 300 ml 30% hydrogen peroxide over 1 hr. The mixture was heated at 40°C for 3 hr. After cooling, the mixture was extracted with diethyl ether and this extract was dried over magnesium sulfate. Evaporation of the solvent and purification of the residue by silica gel flash chromatography using 5% ethyl acetate-hexane gave 56 g of (S)-3-cyclohexyl-1-propyn-3-ol, which by nmr analysis was shown to be 90% e.e. Recrystallization from hexane gave 45 g of the pure S-isomer, mp 56-58 °C, $[\alpha]_D^{25}$ = 11.1° (C = 0.53. $Et_2O$).

Preparation 4

Preparation of (S)-3-Tert-butyldimethylsilyoxy-3-cyclohexylprop-1-yne

To a solution of (S)-3-cyclohexyl-1-propyn-3-ol, (2.76 g, 0.02 mol), in 10 ml N,N-dimethylformamide (DMF), cooled to 0°C, was added imidazole (2.1 g), followed by tert-butyldimethylchlorosilane (3.1 g, 0.02 mol). The mixture was stirred for 3 h. Water (80 ml) and hexane (80 ml) were added; the organic layer was separated and combined with 2 x 80 ml of hexane extractions of the aqueous layer. The solvent was removed (in vacuo), after drying over sodium sulfate, to give a crude residue (4.3 g) which was chromatographed on silica gel (80 g), eluting with ethyl acetate-hexane (2:1, v/v) to afford (S)-3-tert-butyldimethylsilyloxy-3-cyclohexylprop-1-yne.

EXAMPLE 1

Preparation of (1S,6R)-8,8-dichlorobicyclo[4.2.0]oct-2-en-7-one and (1R,6S,7S)-8,8-dichlorobicyclo[4.2.0]oct-2-en-7-ol, the compounds of formula (4) and (5)

A. A mixture of 3 liters of water, 150 g of an active Bakers Yeast, 15 g of edible yeast and 10 g of sucrose were stirred at 32°C. A solution of 24 g of 8,8-dichloro-bicyclo[4.2.0]oct-2-en-7-one in 170 ml of ethanol was added dropwise over a 15 minute period, followed by a further 5 g of sucrose. After stirring for 45 minutes the reaction mixture was centrifuged to remove the yeast, which was washed with acetone and the washings combined with the aqueous product from the centrifuge. The combined product was washed several times with ethyl acetate, the organic washings combined, filtered and the solvent removed from the filtrate under reduced pressure. The residue was chromatographed on silica gel, eluting with 5% acetone in hexane, initially and gradually increasing the proportion of acetone to 100%, giving 4.0 g of (1S,6R)-8,8-dichorobicyclo[4.2.0]oct-2-en-7-one (4) as a liquid, $[\alpha]_D^{25}$ = 78.7° (C = 0.8, $CHCl_3$), and 8.8 g of crude (1R,6S,7S)-8,8-dichlorobicyclo[4.2.0]oct-2-en-7-ol (5), which was recrystallized from hexane to give 6.4 g of pure (5), m.p. = 86-87°C, $[\alpha]_D^{25}$ = -217.1° (C = 0.6, $CHCl_3$).

In an alternative work-up procedure to that shown above, an equal volume of acetone was added to the fermentation broth, the solid filtered off through celite, the celite washed with acetone and solvent removed from the filtrate under reduced pressure. The residue was then chromatographed and purified as shown above.

B. Similarly, following the procedure of paragraph 1.A. above, but starting with the compounds of formula (3) where X is hydrogen or bromo and Y is bromo, the following compounds of formula (4) and (5) are prepared: (1S,6R)-8-bromobicyclo[4.2.0]oct-2-en-7-one, and (1R,6S,7S)-8-bromobicyclo[4.2.0]oct-2-en-7-ol (1S,6R)-8,8-dibromobicyclo[4.2.0]oct-2-en-7-one, and (1R,6S,7S)-8,8-dibromobicyclo[4.2.0]oct-2-en-7-ol.

EXAMPLE 2

Preparation of (1S,6R,7R)-8,8-dichlorobicyclo (4.2.0]oct-2-en-7-ol, the compound of formula (6)

A. A solution of 3.0 g of (1S,6R)-8,8-dichlorobicyclo [4.2.0]oct-2-en-7-one, the compound of formula (4), is dissolved in 120 ml of methanol and treated at 0°C with 1.2 g of sodium borohydride, and the mixture is stirred at 25°C for 1 hour. the solvent is removed under reduced pressure and the residue partitioned between methylene chloride and water. The extract is dried over anhydrous sodium sulfate, filtered and the solvent removed under reduced pressure. The residue is recrystallized from hexane to give (1S,6R,7R)-8,8-dichlorobicyclo[4.2.0]oct-2-en-7-ol, $[\alpha]_D^{25}$ = +214.4° (C = 0.5, $CHCl_3$).

B. Similarly, following the procedure in paragraph 2.A. above, but substituting other ketones from Example 1 for (1S,6R)-8,8-dichlorobicyclo[4.2.0]oct-2-en-7-one, the following compounds of formula (6) are prepared: (1S,6R,7R)-8,8-dibromobicyclo[4.2.0]oct-2-en-7-ol (1S,6R,7R)-8-bromobicyclo[4.2.0]oct-2-en-7-ol

EXAMPLE 3

Preparation of (1R,6S)-8,8-dichlorobicyclo[4.2.0]oct-2-en-7-one, the compound of formula (7)

A. A mixture of 3.9 g of (1R,6S,7S)-8,8-dichlorobicyclo [4.2.0]oct-2-en-7-ol, 9.0 g of pyridinium chlorochromate and 14 g of magnesium sulfate in 200 ml of methylene chloride under nitrogen was refluxed for 4 hours. The mixture was cooled, filtered first through celite then Florisil. Solvent was removed from the eluate under reduced pressure and the residue distilled under vacuum to give 3.1 g of (1R,6S)-8,8-dichlorobicyclo[4.2.0]oct-2-en-7-one, $[\alpha]_D^{25}$ = +71.4°, (C = 0.7, $CHCl_3$).

B. Similarly, starting from (1S,6R,7R)-8,8-dichlorobicyclo[4.2.0]oct-2-en-7-ol, and following the procedure of paragraph 3.A. above, (1S,6R)-8,8-dichlorobicyclo[4.2.0]oct-2-en-7-one is prepared.

C. Similarly, following the procedure in paragraph 3.A. above, but starting with the compounds of formula (5) or (6), the following compounds of formula (4) and (7) are prepared:
(1S,6R)-8,8-dibromobicyclo[4.2.0]oct-2-en-7-one
(1S,6R)-8-bromobicyclo[4.2.0]oct-2-en-7-one
(1R,6S)-8,8-dibromobicyclo[4.2.0]oct-2-en-7-one
(1R,6S)-8-bromobicyclo[4.2.0]oct-2-en-7-one.

EXAMPLE 4

Preparation of (1R,6S)-bicyclo[4.2.0]oct-2-en-7-one

A. To a solution of 1.1 g (1R,6S)-8,8-dichlorobicyclo[4.2.0]oct-2-en-7-one in 10 ml of glacial acetic acid was added 2.0 g of zinc dust in portions over a period of 15 minutes. The mixture was then stirred at 65°C for 1 hour, then a further 0.5 g of zinc dust was added and the mixture stirred at 75°C for 1 hour. The mixture was cooled, filtered, the solid washed with hexane, the filtrate and combined washings diluted with water and extracted with hexane. The hexane layer was dried over anhydrous sodium sulfate, the solvent removed under reduced pressure and the residue distilled under vacuum to give 500 mg of (1R,6S)-bicyclo-[4.2.0]oct-2-en-7-one, the compound of formula (2), $[\alpha]_D^{25}$ = + 171.4°, (C = 0.5, $CHCl_3$).

B. Similarly, starting from (1R,6S)-8-bromobicyclo[4.2.0]oct-2-en-7-one or (1R,6S)-8,8-dibromobicyclo-[4.2.0]oct-2-en-7-one and following the procedure of paragraph 4.A. above, the compound of formula (2) is prepared.

C. Similarly, starting from (1S,6R)-8,8-dichlorobicyclo[4.2.0]oct-2-en-7-one or (1S,6R)-8,8-dibromobicyclo[4.2.0]oct-2-en-7-one or (1S,6R)-8-bromobicyclo[4.2.0]oct-2-en-7-one, and following the procedures of paragraph 4.A. above, the corresponding compound of formula (1) is made, (1S,6R)-bicyclo-[4.2.0.]oct-2-en-7-one.

Example 5

Preparation of Calcium (Z)-(3'S,1S,2S,3R,6S)-4-[2-(3'-hydroxy-3'-cyclohexyl-prop-1'-ynyl)-3-hydroxy-bicyclo-[4.2.0]oct-7-ylidene]butyrate

A. Preparation of (1R,6S)-spiro[bicyclo[4.2.0]oct-2-ene-7,2'-[1.3]dioxolan], (12)

A mixture of 6.4 g of (1R,6S)-bicyclo[4.2.0]oct-2-en-7-one (2), 18.62 g ethylene glycol, 100 ml benzene, and 25 mg p-toluenesulfonic acid is heated at reflux for 4 hr using a Dean-Stark trap to effect continuous removal of water. The cooled reaction mixture is poured onto 100 ml saturated sodium bicarbonate solution and the resulting mixture is extracted with three 75 ml portions of diethyl ether. The combined organic extract is washed with 100 ml saturated sodium chloride solution and is then dried over sodium sulfate. The solvent is removed under vacuum and the residue is distilled under vacuum to give 7.12 g of (1R,6S)-spiro-[bicyclo[4.2.0]oct-2-ene-7,2'-[1.3]dioxolan] (12).

B. Preparation of (1S,2S,4R,7S)-Spiro[3-oxatricyclo-[5.2.0.0$^{2,4}$]nonane-8,2'-[1.3]dioxolan]

To a stirred solution of 5 g of (1R,6S)-spiro[bicyclo[4.2.0]oct-2-ene-7,2'-[1.3]dioxolan], in 40 ml acetone and 20 ml water at 0°C is added 4.76 g of N-bromacetamide over 1 hr. This mixture is stirred at room temperature for 20 hr. To this solution is added 12.4 g potassium carbonate and the resulting mixture is stirred at room temperature for 3 days. The mixture is saturated with sodium chloride and the resulting mixture is extracted with four 150 ml portions of diethyl ether. The combined organic extract is washed with 100 ml of saturated sodium chloride solution and is dried over sodium sulfate. Removal of solvent in vacuum and chromatographic purification of the residue on silica gel eluting with 15% ethyl acetate-hexane gives 3.45 g of a ca. 4:1 mixture of (1S,2S,4R,7S)-spiro[3-oxatricyclo[5.2.0.0$^{2,4}$]nonane-8,2'-[1.3]dioxolan] (15) and (1S,2R,4S,7S)-spiro[3-oxatricyclo[5.2.0.0$^{2,4}$]-nonane-8,2'-[1.3]dioxolan] (16).

C. Preparation of (3'S,1R,2S,3R,6S)-Spiro[2-[3'-t-butyldimethylsilyloxy-3'-cyclohexylprop-1'-ynyl)-3-hydroxybicyclo[4.2.0.]octane-7,2'-[1.3]dioxolane] (19)

To a mixture of 21 g (S)-3-t-butyldimethylsilyloxy-3-cyclohexyl-1-propyne in 100 ml tetrahydrofuran at 0°C under argon atmosphere was added over 20 min 60 ml 1.26 M n-butylithium in hexane. The resulting solution was cooled to -78°C and a solution of 5.5 g of (1S,2S,4R,7S)-spiro[3-oxatricyclo[5.2.0.0$^{2,4}$]-nonane-8,2'-[1.3]dioxolan] in 25 ml tetrahydrofuran was added. To this stirred mixture at -78°C was added 2.5 ml boron trifluoride etherate dropwise over a 25 min period. To this mixture was added 25 ml saturated sodium sulfate solution. The resulting mixture was warmed to room temperature and it was extracted thoroughly with ethyl acetate. This solution was dried over sodium sulfate and was concentrated in vacuo to give an oily residue. Volatiles were removed on a Kugelrohr distillation device at 95°C (0.1 mm Hg) to leave 9.28 g of a residue, which was further purified by flash chromatography on silica gel using 1.8% acetone-dichloromethane. This procedure gave 5.87 g of a mixture of the title compound as an oil: CIMS m/z 452 (M + $NH_4^+$). Calcd. for $C_{25}H_{42}SiO_4$: C, 69.08, H, 9.74. Found: C, 69.22; H, 9.97.

D. Preparation of (3'S,1R,2S,3R,6S)-2-(3'-hydroxy-3'-cyclo-hexylprop-1'-ynyl)-3-hydroxybicyclo[4.2.0]octan-7-one (21)

A solution of 182 mg of the (3'S,1R,2S,3R,6S)-cyclohexylpropynyl acetal (19), 1 ml of acetonitrile, 0.2 ml of water and 0.2 ml of 2 N sulfuric acid was stirred at ambient temperature for 16 hours. The reaction was quenched by neutralization with aqueous sodium bicarbonate and the mixture was extracted with diethyl ether. The extracts were dried with magnesium sulfate, evaporated to dryness and the residue was purified by short column silica-gel chromatography. Elution with ethyl acetate-hexane (7:3), gave 94 mg (3'S,1R,2S,3R,6S)-2-(3'-hydroxy-3'-cyclohexylprop-1'-ynyl)-3-hydroxybicyclo[4.2.0]octan-7-one, m.p. 93-98°C.

E. Preparation of (Z)-(3'S,1S,2S,3R,6S)-4-[2.-(3'-hydroxy-3'-cyclohexylprop-1'-ynyl)-3-hydroxybicyclo[4.2.0]-oct-7-ylidene]butanoic acid (8)

A stock solution of dimsyl sodium was prepared by dissolving 1.56 g sodium hydride in 65 ml dimethyl sulfoxide at 65°C under nitrogen. To a stirred slurry of 2.06 g of 3-carboxypropyltriphenylphosphonium bromide in 10 ml dimethyl sulfoxide under nitrogen was added 9.4 ml of the stock solution of dimsyl sodium. After 20 min at 23°C a solution of 260 mg of (3'S,1R,2S,3R,6S)-2-(3'-hydroxy-3'-cyclohexylprop-1'-ynyl)-3-hydroxybicyclo[4.2.0]octane-7-one in 1 ml of dimethyl sulfoxide was added in one portion. After 4 h at 23°C the mixture was poured onto 15 ml 5% sodium carbonate solution. This mixture was washed with two 30 ml portions of ethyl acetate and was then acidified with conc. HCl. The aqueous layer was extracted three times with 50 ml portions of diethyl ether. The combined ether extract was concentrated to 20 ml and this was kept at -20°C for 2 h. The resulting precipitate was filtered and was discarded. Evaporation of the filtrate gave 430 mg of an oil. This material was purified by silica gel flash chromatography using a solvent mixture of acetic acid-ethyl acetate-hexane (0.25:75:25) to give 337 mg of an oil. Further purification by silica gel flash chromatography using a solvent mixture of acetic acid-methanol-dicloromethane (0.2:5.3:94.5) afforded (Z)-(3'S,1S,2S,3R,6S)-4-[2-(3'-hydroxy-3'-cyclohexylprop-1'-ynyl)-3-hydroxybicyclo-[4.2.0]oct-7-ylidene]butyric acid, 138 mg: $[\alpha]_D^{25}$ +105° (C=0.4, $CHCl_3$). Elemental Analysis: Calcd. C, 72.80; H, 8.73, Found C, 73.00; H, 8.51.

F. Preparation of calcium (Z)-(3'S,1S,2S,3R,6S)-4-[2-(3'-hydroxy-3'-cyclohexyl-prop-1'-ynyl)-3-hydroxybicyclo-[4.2.0]-oct-7-ylidene]butyrate

96 mg of (Z)-(3'S,1S,2S,3R,6S)-4-[2-(3'-hydroxy-3'-cyclohexyl-prop-1'-ynyl)-3-hydroxybicyclo-[4.2.0]-oct-7-ylidene]butyric acid and 7.01 mg of calcium oxide were mixed in 1.5 ml of water and 1.2 ml of tetrahydrofuran. The mixture was heated at 50°C for 30 minutes and filtered. The residue was dissolved in 1.2 ml of tetrahydrofuran and then added to 15 ml of ether. The precipitate was stirred at room temperature and then filtered. The solid was washed with anhydrous ether and dried under high vacuum at room temperature overnight to give 66 mg of the title compound, $[\alpha]_D^{25}$ = 114.0°; mp. 138-143°C.

Example 6

Preparation of (1R,6S,7R)-bicyclo[4.2.0]oct-2-en-7-ol (5B)

To a solution of 320 mg (1.65 mmol) of (5) and 2 mL (7.5 mmol) of tri-n-butyltin hydride in 10mL of hexane was added 50 mg of azobisisobutyronitrile (AIBN) under an atmosphere of nitrogen. The mixture was heated at reflux in the presence of light for 16 h. The solvent was removed and the residue purified by flash chromatography with 3% $Et_2O/CH_2Cl_2$. Final purification was performed by distillation at reduced pressure (1 mm Hg, 60-70 °C) to afford 100 mg (49% yield) of the title compound: $[\alpha]_D^{25}$ = 121.4 ° (C = 0.52, $CHCl_3$).

The compound of the formula (5B) can be used to prepare a compound of the fomula (2) in accordance with reaction conditions described in Example 3. In turn, the compound of the formula (2), in accordance with the reaction conditions of Example 5, can be used to prepare the calcium salt of the compound of the formula (8).

**Claims**

1. A process for preparing a bicyclo[4.2.0]octane derivative, which process comprises: contacting a racemic or non-racemic mixture of a bicyclo[4.2.0]octane derivative of the formula

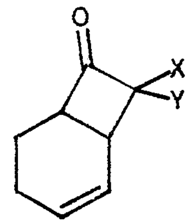

(3)

wherein X is hydrogen or bromo when Y is bromo, or X is chloro when Y is chloro, with a micro-organism of a preparation derived from a micro-organism under conditions sufficient to give a mixture of compounds represented by the formulas

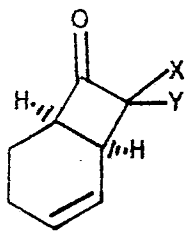

and

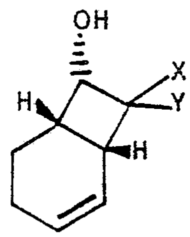

(4) (5)

wherein X and Y have the above meanings.

2. The process of Claim 1 wherein said bicyclo[4.2,0]octane derivative is a compound of the formula (1) or (2).

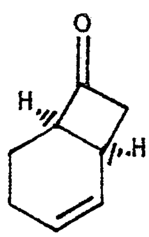

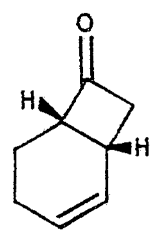

(1) (2)

3. The process of Claim 1 wherein the micro-organism is Baker's Yeast.

4. The process of Claim 1, 2 or 3 which further comprises:

(a) separating the compound represented by the formula (4) from the compound represented by the formula (5) and

(b) optionally recrystallizing the compound of the formula (5).

5. The process of Claim 4 which further comprises:

(a) dehalogenating a compound represented by the formula (5) to a compound represented by the formula

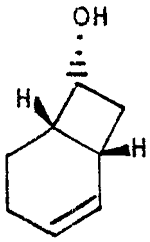

(5B)

and optionally

(b) oxidizing a compound represented by the formula (5B) to a compound represented by the formula (2).

6. The process of Claim 4 wherein X and Y are each chloro.

7. The process of Claim 1 or 6 which further comprises contacting the compound represented by the formula (4) with a mild reducing agent to give a compound represented by the formula:

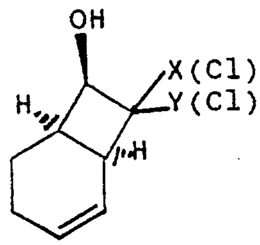

(6)

wherein X and Y have the above meanings (preferably chloro) and (b), optionally recrystallizing the compound represented by the formula (6) from a suitable solvent.

8. The process of Claim 7 wherein the reducing agent is sodium borohydride.

9. The process of Claim 7 which further comprises oxidation of the compound represented by the formula (6) to a compound represented by the formula (4).

10. The process of Claim 9 wherein the oxidant is a solution of chromic acid in sulfuric acid, sodium dichromate or an organic chromium reagent such as pyridinium chlorochromate.

11. The process of Claim 1 or 2 or 6 or 9 which further comprises dehalogenating (dechlorinating) the compound represented by the formula (4) to give a compound represented by the formula:

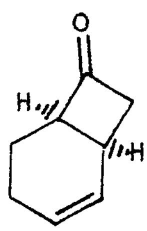

(1)

12. The process of Claim 11 wherein the dehalogenation (dechlorination) is carried out with zinc in acetic acid.

13. The process of Claim 1 or 6 which further comprises oxidizing the compound represented by the formula (5) to form a compound represented by the formula:

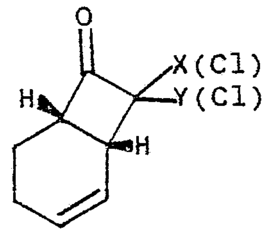

(7)

wherein X and Y have the above meanings (preferably chloro).

14. The process of Claim 13 wherein the oxidant is chosen from a solution of chromic acid in sulfuric acid, sodium dichromate or an organic chromium reagent such as pyridinium chlorochromate.

15. The process of Claim 13 or 14 which further comprises dehalogenating (dechlorinating) the compound represented by the formula (7) to give a compound represented by the formula:

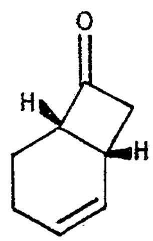

(2)

16. The process of Claim 15 wherein the dehalogenation (dechlorination) is carried out with zinc in acetic acid.

17. A compound represented by the formula:

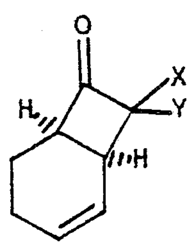

(4)

wherein X is hydrogen or bromo when Y is bromo, or X is chloro when Y is chloro, preferably the compound (1S,6R)-8,8-dichlorobicyclo[4.2.0]oct-2-en-7-one.

18. A compound represented by the formula:

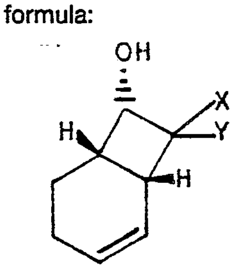

(5)

wherein X is hydrogen or bromo when Y is bromo, or X is chloro when Y is chloro, preferably the compound (1R,6S,7S)-8,9-dichlorobicyclo[4.2.0]-oct-2-en-7-ol.

19. A compound represented by the formula:

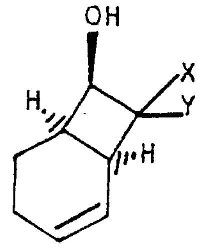

(6)

wherein X is hydrogen or bromo when Y is bromo, or X is chloro when Y is chloro, preferably the compound (1S,6R,7R)-8,8-dichlorobicyclo[4.2.0]oct-2-en-7-ol.

20. A compound represented by the formula:

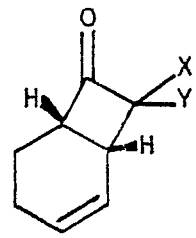

(7)

wherein X is hydrogen or bromo when Y is bromo, or X is chloro when Y is chloro, preferably the compound (1R,6S)-8,8-dichlorobicyclo[4.2.0]oct-2-en-7-one.

21. A compound represented by the formula:

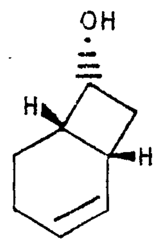

(58)

namely, the compound (1R,6S,7R)-bicyclo[4.2.0]oct-2-en-7-ol.

22. The process of Claims 1 to 16 or 25 wherein said bicyclo[4.2.0]octane derivatives are repesented by the formula:

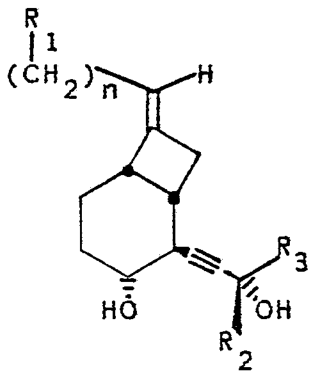 or 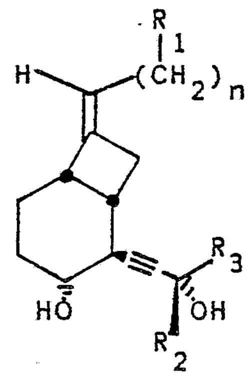 or 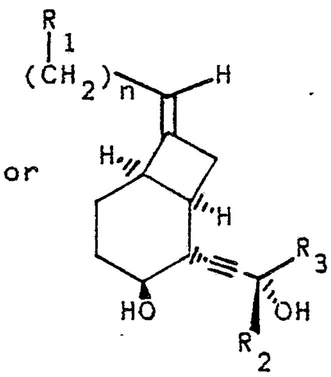

(8) (9) (10)

or the (E)-isomer of (10) and the pharmaceutically acceptable, non-toxic salts thereof wherein:
n is 2 or 3;
$R_1$ is $CH_2OH$, CHO, $CO_2R$ or $CO_2H$;
$R_2$ is hydrogen or methyl; and
$R_3$ is linear or branched alkyl having 5-10 carbon atoms,

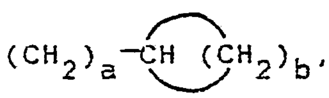, 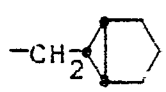, 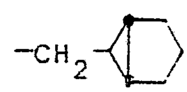, or $-(CH_2)_m$-phenyl

optionally substituted with lower alkyl, lower alkoxy, trifluoromethyl, or halogen,
in which
a is 0, 1 or 2;
b is 3-7;
m is 1 or 2; and
R is

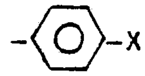

wherein X is
$-\overset{O}{\overset{\|}{C}}-CH_3$, $-NH\overset{O}{\overset{\|}{C}}CH_3$, $-\overset{O}{\overset{\|}{C}}-C_6H_5$, $-NH\overset{O}{\overset{\|}{C}}-C_6H_5$, or
$-NH\overset{O}{\overset{\|}{C}}N(R_4)_2$;
in which each $R_4$ is independently hydrogen or lower alkyl having 1-6 carbon atoms which process comprises the following additional steps:

(a) protecting the carbonyl group of an enantiomeric compound of the formula (1) or (2) to give a compound of the formula:

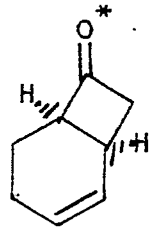

or

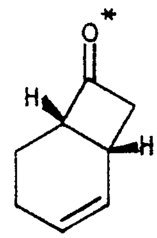

(11) (12)

wherein the asterisk signifies that the carbonyl group is protected;

(b) epoxidizing the compound of formula (11) or (12) to give compounds of the formulas:

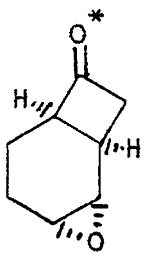

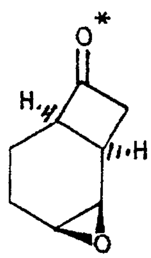

or

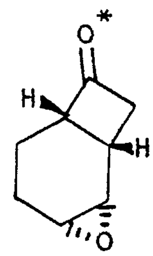

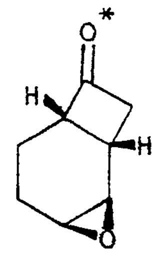

(13) (14) (15) (16)

as a mixture of (13) and (14) or a mixture of (15) and (16), respectively;

(c) treating the mixture of epoxides with a lithium acetylenic reagent to give a compound of the formula:

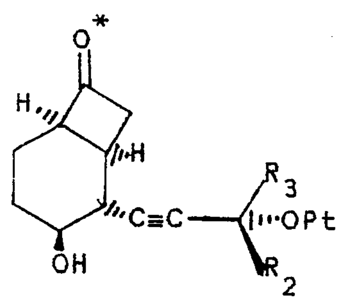

or

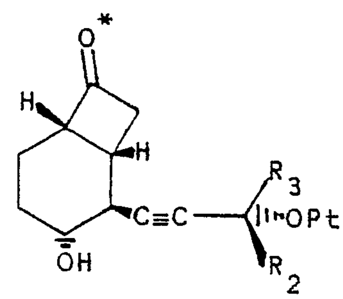

(18) (19)

wherein Pt is a protecting group;

(d) removing the protecting groups of formula (18) or (19) to give a compound of the formula:

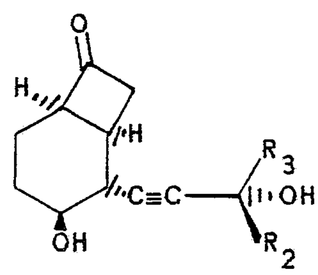 or 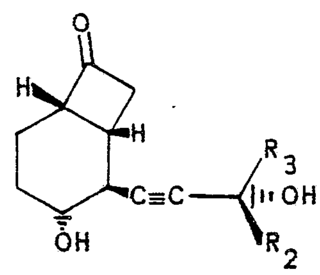

(20) (21)

(e) reacting the compounds of formulas (20) or (21) with a phosphorus ylid (Wittig reagent) to give a mixture of the compounds of formulas (8) and (9) or (10) and the (E)-isomer corresponding to (10); and

(f) separating the individual isomers of (8) or (9) or (10) and its (E)-isomer, and optionally converting a compound of the formula (8) or (9) or (10) or its (E)-isomer wherein $R_1$ is COOH to a pharmaceutically acceptable, non-toxic salt thereof.

23. The use of the compounds of claims 17 to 21 in the preparation of the bicyclo[4.2.0]octane derivatives, in particular of the formulas (1), (2), (8), (9), (10) and its (E)-isomer.

24. The use of the compounds of formula (1), (2) and (5B) in the preparation of the bicyclo[4.2.0]octane derivatives, in particular of the formulas (8), (9), (10) or its (E)-isomer.

25. A process for the preparation of a compound of the formula:

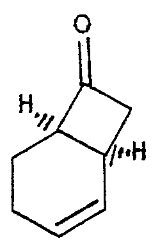 or 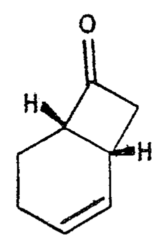

(1) (2)

or bicyclo[4.2.0]octane derivatives on the basis of the compounds of the formula (1) or (2), which process comprises dehalogenating a compound of the formula:

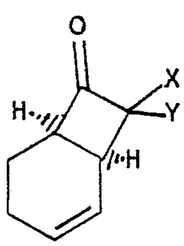 or 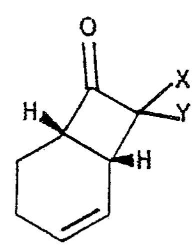

(4) (7)

repsectively,
wherein X and Y have the above meanings.